# EUROPEAN PATENT SPECIFICATION

(11) **EP 4 164 537 B1**
(45) Date of publication and mention of the grant of the patent: **03.12.2025**
(21) Application number: 21737150.9
(22) Date of filing: 08.06.2021
(51) Int. Cl.: A61B 34/30, A61B 90/00

(54) **MULTI-ARM ROBOTIC SYSTEM ENABLING MULTIPORTAL ENDOSCOPIC SURGERY**
MEHRARMIGES ROBOTERSYSTEM FÜR ENDOSKOPISCHE MULTIPORT-CHIRURGIE
SYSTÈME ROBOTIQUE À BRAS MULTIPLES PERMETTANT UNE CHIRURGIE ENDOSCOPIQUE MULTIPORTAIL

(30) Priority: 10.06.2020 US 202063037332 P
(43) Date of publication of application: 19.04.2023
(73) Proprietor: Mazor Robotics Ltd., 3079830 Caesarea (IL)
(72) Inventor: SHMAYAHU, Yizhaq, 3079830 Caesarea (IL); ZEHAVI, Eli, 3079830 Caesarea (IL)
(74) Representative: Viering, Jentschura & Partner mbB Patent- und Rechtsanwälte
(86) International application number: PCT/IB2021/055051
(87) International publication number: WO 2021/250580

(56) References cited:
- US-A1- 2005 096 502
- US-A1- 2011 238 079
- US-A1- 2014 243 849
- US-A1- 2019 125 457
- CHOI CHANG MYONG ET AL: "How I do it? Biportal endoscopic spinal surgery (BESS) for treatment of lumbar spinal stenosis", ACTA NEUROCHIRCA, SPRINGER VERLAG, AT, vol. 158, no. 3, 18 January 2016 (2016-01-18), pages 459 - 463, XP035887988, ISSN: 0001-6268, [retrieved on 20160118], DOI: 10.1007/S00701-015-2670-7

## Description

### FIELD

Embodiments of the present disclosure relate to the field of endoscopic surgery, especially for use in minimally invasive robotic operations to decompress spinal stenosis and achieve fusion of vertebrae.

### BACKGROUND

Many people undergo surgery for spinal stenosis each year. Endoscopic spine surgery for patients suffering from this condition allows percutaneous decompression under local anesthesia. The development of minimally invasive alternatives to operations that in the past entailed significant tissue damage and blood loss has major benefits. Endoscopic approaches to minimally invasive surgery (MIS) have opened access to surgical treatment for older and frailer patients. In addition, endoscopic MIS significantly shortens the requirement for operating room time for all patients; supports shorter hospital stays and reduces surgical blood loss; and provides a patient reported alternative to neuromonitoring, enhancing safety. Since the patient can remain awake, the patient is able to report pain or sensory disturbances caused by tool maneuvers in excessive proximity to spinal nerves or to the dorsal root ganglia.

However, the MIS approach to spinal decompression also has drawbacks which may limit the use of this technology. For example, use of a single endoscope port with multiple channels restricts the size of the port carrying the tool or other end effector to a few millimeters in diameter. Such miniaturized tools are inadequate to properly perform procedures such as spinal decompression and spinal fusion. Using a second endoscopic arm with a single channel allows the use of a larger tool; the drawback of this approach is the difficulty of coordinating the operation of the tools within a patient when the two arms are manipulated by the surgeon who lacks direct visibility of the surgical field.

Related applications commonly assigned to the present applicant include:
US 9,125,556 Robotic Guided Endoscope
PCT/IB2019/058795 Versatile Multi-Arm Robotic Surgical System
US 62/952,958 A Multi-Arm Robotic System Enabling Endoscopic Ultrasound Guidance for Spine Surgery
US 2011/238079 A1 and US 2019/125457 A1 describe a system for endoscopic surgery, comprising: a first robotic arm with an endoscopic camera carried thereon, a second robotic arm with a surgical end effector carried thereon, an irrigation nozzle configured to inject fluid into a preselected tissue region, and at least one controller configured to control at least the first robotic arm and the second robotic arm, such that the pose of the endoscopic camera and the pose of the surgical end effector are known to the controller.

### SUMMARY

The present invention provides a system for endoscopic surgery with the features according to claim 1. Further preferred embodiments are described in the dependent claims.

The details of one or more aspects of the disclosure are set forth in the accompanying drawings and the description below. Other features, objects, and advantages of the techniques described in this disclosure will be apparent from the description and drawings, and from the claims.

The phrases "at least one", "one or more", and "and/or" are open-ended expressions that are both conjunctive and disjunctive in operation. For example, each of the expressions "at least one of A, B and C", "at least one of A, B, or C", "one or more of A, B, and C", "one or more of A, B, or C" and "A, B, and/or C" means A alone, B alone, C alone, A and B together, A and C together, B and C together, or A, B and C together. When each one of A, B, and C in the above expressions refers to an element, such as X, Y, and Z, or class of elements, such as X₁-Xₙ, Y₁-Yₘ, and Z₁-Zₒ, the phrase is intended to refer to a single element selected from X, Y, and Z, a combination of elements selected from the same class (e.g., X₁ and X₂) as well as a combination of elements selected from two or more classes (e.g., Y₁ and Zₒ).

The term "a" or "an" entity refers to one or more of that entity. As such, the terms "a" (or "an"), "one or more" and "at least one" can be used interchangeably herein. It is also to be noted that the terms "comprising", "including", and "having" can be used interchangeably.

The preceding is a simplified summary of the disclosure to provide an understanding of some aspects of the disclosure. This summary is neither an extensive nor exhaustive overview of the disclosure and its various aspects, embodiments, and configurations. It is intended neither to identify key or critical elements of the disclosure nor to delineate the scope of the disclosure but to present selected concepts of the disclosure in a simplified form as an introduction to the more detailed description presented below. As will be appreciated, other aspects, embodiments, and configurations of the disclosure are possible utilizing, alone or in combination, one or more of the features set forth above or described in detail below.

Numerous additional features and advantages of the present invention will become apparent to those skilled in the art upon consideration of the embodiment descriptions provided hereinbelow.

### DEFINITIONS

*Medical image segmentation* is the process of automatic or semi-automatic detection of boundaries within a 2D or 3D image.

*Surgical end effector* is any implement used on a robotic arm in the process of carrying out a surgical procedure; typically, a drill, scalpel, irrigation device, cautery, ablation head, or other surgical tool.

### BRIEF DESCRIPTION OF THE DRAWINGS

Embodiments of the present disclosure will be understood and appreciated more fully from the following detailed description, taken in conjunction with the drawings in which:
Fig.1 illustrates a schematic representation of an exemplary system of the present disclosure incorporating a number of robotic arms;
Fig. 2 is an axial schematic view across a spine, showing various access paths for performance of surgical procedures described in this disclosure, which can be performed using the system of Fig. 1;
Fig. 3 is a flow chart showing the steps followed in an exemplary implementation of a surgical procedure carried out by the system of claim 1;
Fig. 4 illustrates schematically how an intraoperative image acquired by the endoscopic camera may be superimposed on a preoperative MRI or CT image of the surgical field; and
Fig. 5 shows a block diagram of the structure of the control components of a typical embodiment of the systems of this disclosure.

### DETAILED DESCRIPTION

It should be understood that various aspects disclosed herein may be combined in different combinations than the combinations specifically presented in the description and accompanying drawings. It should also be understood that, depending on the example or embodiment, certain acts or events of any of the processes or methods described herein may be performed in a different sequence, and/or may be added, merged, or left out altogether (e.g., all described acts or events may not be necessary to carry out the disclosed techniques according to different embodiments of the present disclosure). In addition, while certain aspects of this disclosure are described as being performed by a single module or unit for purposes of clarity, it should be understood that the techniques of this disclosure may be performed by a combination of units or modules associated with, for example, a computing device and/or a medical device.

In one or more examples, the described methods, processes, and techniques may be implemented in hardware, software, firmware, or any combination thereof. If implemented in software, the functions may be stored as one or more instructions or code on a computer-readable medium and executed by a hardware-based processing unit. Alternatively or additionally, functions may be implemented using machine learning models, neural networks, artificial neural networks, or combinations thereof (alone or in combination with instructions). Computer-readable media may include non-transitory computer-readable media, which corresponds to a tangible medium such as data storage media (e.g., RAM, ROM, EEPROM, flash memory, or any other medium that can be used to store desired program code in the form of instructions or data structures and that can be accessed by a computer).

Instructions may be executed by one or more processors, such as one or more digital signal processors (DSPs), general purpose microprocessors (e.g., Intel Core i3, i5, i7, or i9 processors; Intel Celeron processors; Intel Xeon processors; Intel Pentium processors; AMD Ryzen processors; AMD Athlon processors; AMD Phenom processors; Apple A10 or 10X Fusion processors; Apple A11, A12, A12X, A12Z, or A13 Bionic processors; or any other general purpose microprocessors), graphics processing units (e.g., Nvidia GeForce RTX 2000-series processors, Nvidia GeForce RTX 3000-series processors, AMD Radeon RX 5000-series processors, AMD Radeon RX 6000-series processors, or any other graphics processing units), application specific integrated circuits (ASICs), field programmable logic arrays (FPGAs), or other equivalent integrated or discrete logic circuitry. Accordingly, the term "processor" as used herein may refer to any of the foregoing structure or any other physical structure suitable for implementation of the described techniques. Also, the techniques could be fully implemented in one or more circuits or logic elements.

Before any embodiments of the disclosure are explained in detail, it is to be understood that the disclosure is not limited in its application to the details of construction and the arrangement of components set forth in the following description or illustrated in the drawings. The disclosure is capable of other embodiments and of being practiced or of being carried out in various ways. Also, it is to be understood that the phraseology and terminology used herein is for the purpose of description and should not be regarded as limiting. The use of "including," "comprising," or "having" and variations thereof herein is meant to encompass the items listed thereafter and equivalents thereof as well as additional items. Further, the present disclosure may use examples to illustrate one or more aspects thereof. Unless explicitly stated otherwise, the use or listing of one or more examples (which may be denoted by "for example," "by way of example," "e.g.," "such as," or similar language) is not intended to and does not limit the scope of the present disclosure.

The terms proximal and distal are used in this disclosure with their conventional medical meanings, proximal being closer to the operator or user of the system, and further from the region of surgical interest in or on the patient, and distal being closer to the region of surgical interest in or on the patient, and further from the operator or user of the system. Surgical procedures/methods described in the following do not form part of the invention but are helpful in understanding the invention.

Embodiments of the present disclosure provide new exemplary systems and methods which overcome at least some of the disadvantages of prior art systems and methods in the field of endoscopic spine surgery. Some embodiments of this disclosure describe a robotically-controlled, multi-arm surgical system with at least one robotic arm controlling the motion of an endoscope having a channel with an optical camera and an irrigation device, and another robotic arm controlling one or more end effectors such as a surgical tool, for performing spinal procedures with minimal tissue damage and under conditions lacking direct visualization of the operative field by the surgeon. An end effector carrying a surgical tool is termed in this disclosure "a surgical end effector". Additional robotic arms and endoscopes may be added as needed for specific operations. Such a configuration, with multiple robotic arms controlled by a single controller that identifies and stores the coordinate systems of each arm, allows for the performance of internal procedures with a self-generated, virtual field of view, as opposed to direct observation. Embodiments of the present disclosure have an advantage over a single endoscopic arm with multiple channels, in that a single-arm configuration is limited in the size of the surgical tool that can be inserted through such a port. Having multiple endoscopic ports allows the use of conventional surgical tools rather than the smaller ones required for a single, multichannel endoscopic port. In addition, embodiments of the methods in the present disclosure enable endoscopic visualization of the surgical field within the solid tissue of the dorsal spinal area, by the generation of a series of fluid-filled cavities in which an imaging device may be inserted endoscopically.

In contrast to some systems and methods of endoscopic surgery using ultrasound visualization of the surgical field, embodiments of the present disclosure use optical visualization of the operative field. Several relevant differences in the two methods of visualization provide distinct advantages in the field of endoscopic surgery. Because the spine has no natural cavity, traditional endoscopic visualization of a naturally existing space is not possible. Endoscopic ultrasound provides the advantage of depth correlation between the two robotic arms, one holding the ultrasound probe and the other holding a surgical tool. However, ultrasound visualization is challenged by the fact that both the probe and the tool generally need to be in approximately the same two-dimensional plane. For ultrasound imaging, the probe must be in contact with the tissue to be imaged, without an intervening cavity. In contrast, a camera using optical visualization typically relies on an intervening cavity to image a three-dimensional surface. An optical camera has the advantage that it avoids the limitation of being confined to a single two-dimensional plane, in that the camera may image the three-dimensional surface of an internal cavity. Since in the absence of an intervening space between the tissue being imaged and the camera, optical images typically cannot be generated, a frequent task in optical imaging is to generate a cavity within the tissue to allow the camera to acquire surface images. For this reason, spinal endoscopy has lagged behind endoscopic procedures performed on hollow organs which have an existing cavity. Because creation of the cavity often causes a distortion in the tissue, a main challenge of this step is how to create a field of vision that corresponds to identifiable anatomical structures. A single cavity is used for both the endoscope housing the camera with a light source and the means of irrigation, and for the surgical end effector held by the second robotic arm. In some embodiments, infrared imaging or echo waves may be used as adjunct measures to identify specific anatomical features such as blood vessels.

For endoscopic spinal decompression and spinal fusion operations, larger tools are sometimes needed that cannot be inserted through a single 5-7mm endoscope. Some of the systems described in the current disclosure, having two endoscopic ports, allow for one port to be dedicated just to the surgical end effector, thus enabling the use of larger tools. Whereas endoscopic surgery has on average a more rapid recovery rate than open procedures, performing a more complicated procedure endoscopically is highly challenging. It may require many fluoroscopic images to get the right angle and geometry, thus exposing the patient to higher levels of radiation. More complex operations performed endoscopically often require the biportal access described in the present disclosure. Biportal access with coordinated guidance of the two ports by the robotic control system allows procedures to be performed with less training, allows the use of larger tools, and reduces radiation from multiple fluoroscopy. This approach, as described in embodiments below, involves identifying the tissue planes, predicting where the cavity will form between the tissue planes, and subsequently creating the cavity.

In some embodiments, a presurgical plan designed to accomplish the desired surgical outcome is input to the system for carrying out the method. The plan includes a general outline of the steps, such as creating an incision, opening the surrounding muscles, cutting the vertebral lamina, eliminating a bone spur, removing a herniated disc, and closing the muscle and skin. In order to carry out these steps, the system plans the initial placement of the surgical end effector and the endoscopic camera. A limitation of planning each step in detail is that patients who require surgery often have atypical anatomy, which contributes to their discomfort and necessitates surgical intervention. Thus, personalization of each procedure is an important and helpful aspect of this system, because it uses a registration process based on a preoperative MRI or CT of the specific individual, whose anatomy is thus known from the preoperative imaging studies. Even though the patient's anatomy in the region of surgical interest is known, the surgeon still faces unknown factors in terms of how the patient's tissue will be distorted by creation of the fluid cavity, and how the remaining tissue in the field of interest will react in response to release of excess pressure by removal of impinging tissue, such as herniated disc or spinal lamina. Thus, the registration process may be repeated at intervals throughout the surgical process, to ensure fidelity and accuracy of the procedure.

While most endoscopic applications make use of an existing anatomic cavity, spine endoscopy typically requires the generation of an endoscopic cavity. In exemplary embodiments of the methods of the present disclosure for spinal endoscopy, three primary potential spaces exist for positioning the endoscopic camera and fluid-filled cavity. These potential spaces are intradiscal, epidural, and periarticular. Removal of the ligamentum flavum may be indicated in operations such as flavectomy for spinal stenosis, in which epidural monitoring is performed. Epidural endoscopic monitoring may also be used for procedures to alleviate foraminal stenosis. A third indication for endoscopic monitoring of a spinal operation is for nerve ablation to relieve facet joint pain, in which the periarticular space is used to create a cavity for monitoring progress of the procedure. Additional implementations of the disclosed methods may be applied to various potential tissue cavities in other areas of the spine and skeletomuscular system.

Initial steps of a typical procedure would be performed by the surgical end effector under robotic control. When the surgical end effector cannot progress further without danger of tissue damage or without visualizing the path forward, the endoscopic camera carried on a second robotic arm is inserted into the patient, typically from a different entry point, between tissue planes. A cavity is formed by injecting a fluid, such as cooled, sterile saline, under regulated pressure in the area of the surgical end effector or tool. The position, shape, and size of the cavity are significant in defining the field of view and change during subsequent stages of the procedure. The endoscopic cavity must be regulated dynamically in order to be maintained for given anatomical structures, tool positions and irrigation parameters. In terms of the required fluid dynamics, the irrigation fluid may be constantly injected typically at ~50mm Hg, depending on the patient's physiology. The injection pressure is typically 15-30 mmHg below diastolic pressure, such that it keeps the fluid-filled cavity open all the time but does not prevent blood flow because of venous compression. The use of cold irrigation fluid reduces bleeding. Once the cavity opens up, the tool can be manipulated into the cavity and then used under direct vision by the camera. Typically, the two ports come from opposite sides of the surgical field; for example, the tool may enter from left and camera from right. Working only in the cavity overcomes the limitation of not having depth of vision. Knowledge of anatomical features below the surface is based on the registration process with the pre-operative MRI or CT image set.

In some prior approaches, endoscopic spine surgery is performed via a single port, providing an unusual, narrow view of the operative field and restricting the surgeon's repertoire to a limited set of predefined tools accommodated by the working channel of the endoscope. These aspects not only limit the extent and type of procedures that can be performed, but require a long training period. Having access to the operative field from more than one position or angle improves the surgeon's ability to view and access the surgical field.

Multi-portal endoscopy allows for the use of standard end effectors, and provides a more effective field of view for the endoscope. Standard tools are often wider than a mono-portal endoscopic channel, since they can be inserted through a port independently of the endoscope viewing elements; they are thus more versatile in function and can increase procedural efficiency. The view of the surgical field in multiport endoscopy is more familiar to surgeons and can facilitate shortening of the learning curve.

However, multiport endoscopy often involves high accuracy in positioning the robotic arm holding the endoscopic camera and the end effector holding the tool - both initially and throughout the procedure - because the surgeon does not have precise knowledge of the position of one endoscopic end effector relative to the other. In monoport endoscopy, both the camera and the surgical end effector are contained in the same port, so their relation to each other is known and fixed. In multiport endoscopy, each port is manipulated independently. If the surgeon is holding and manipulating each port, and the operation is being carried out without direct vision, it requires a great degree of skill to maintain the two ports in alignment or within the same field of view. This is technically challenging and time demanding for most surgeons, and hence used only in highly specialized centers.

In some implementations of the presently disclosed methods, the surgeon, using a multi-arm robotic system of the present disclosure, can calculate initial tracks for all ports and establish the site of cavities for imaging and surgical manipulation. The system can then continuously calculate the optimal positions and orientations for the endoscope to enable it to optimally visualize the working area; and it can aid in interpreting the image observed using its anatomical positioning component.

The robotic system maintains a continuously updated anatomical model, based on pre-operative MRI or CT and current endoscopic image analysis, and holds accurately registered tool positions. Image processing algorithms and databases may be incorporated to allow recognition of anatomical features in both MRI or CT images and in the endoscopic camera-acquired images. A biomechanical model is used to incorporate fluid dynamics utilizing fluid-flow control, image analysis and biomechanical modeling to provide continuous assessment of the size and shape of the cavity generated.

Utilizing endoscopic image analysis, the system or the surgeon may continuously refine the registration, and account for vertebral or soft tissue movement or modification. The path planning is done to ensure that trauma to surrounding tissues, muscles, ligaments and tendons is minimized. In some embodiments, other imaging modalities may be incorporated into the overall imaging of the surgical field. For example, ultrasound, EEG, infrared, or echo flowmetry may be used to locate internal spaces, identify nerves, or find the location of blood vessels.

Important features of certain embodiments discussed herein include: 1) the ability to personalize the surgical approach based on preoperative MRI or CT registration with intraoperative images, 2) the ability to create and maintain from a potential space or preselected tissue region an endoscopic cavity for imaging; 3) iterative intraoperative planning in real time, based on changes in tissue alignment during the course of the operation; and 4) the ability to manipulate the surgical tool within the created endoscopic cavity based on a derived composite field of view from the intra-operative registration. In this manner, these embodiments enable surgeons to offer a surgical solution to patients who would not be capable of undergoing an open operation. In addition, they reduce the cost of a given procedure and improve procedural outcomes.

It is to be understood that references to MRI or CT images or image sets, are only brought as the most commonly used three-dimensional imaging modalities used currently in spinal surgery imaging. However, the apparatus and methods of the present disclosure are not intended to be limited to the use of MRI or CT imaging, but are intended to be used with any suitable three-dimensional imaging technique that may be available.

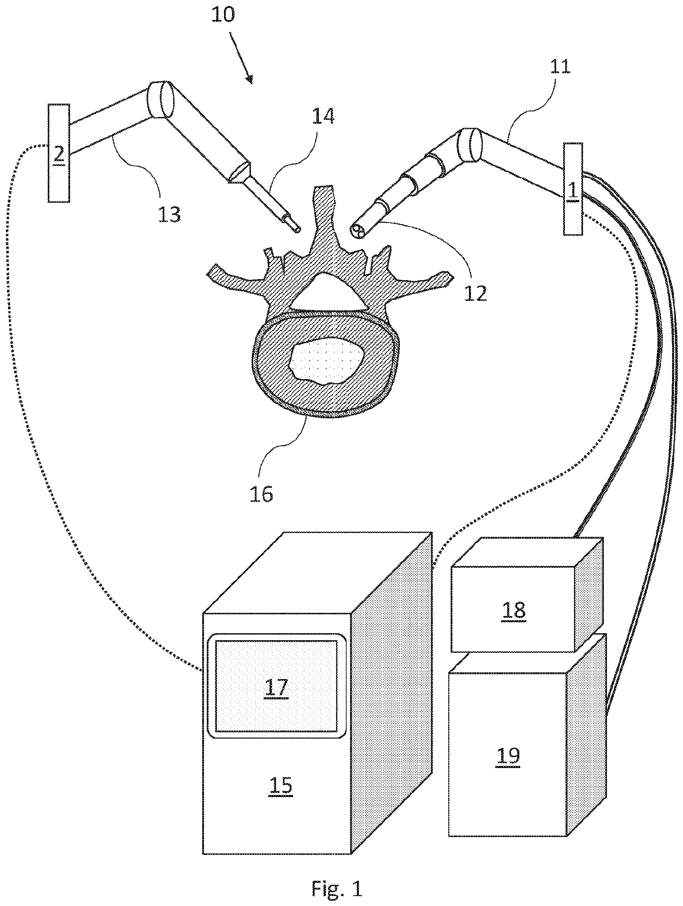

Reference is now made to Fig. 1, which illustrates schematically the structural components of one exemplary system of the present disclosure. A typical embodiment of the system 10 comprises at least two robotically controlled arms 1, 2, fixedly mounted relative to each other, in a convenient location, such as relative to the surgical bed, or on a movable equipment cart or a wall, in some embodiments in a single unit with the controller. Since the mutual positions of the bases of the robots are known, the position and orientation of each robotic arm is known to the controller 15, such that if the pose of one robotic arm is changed by the controller, the pose of the second arm relative to the new pose of the first arm is known to the controller. The control system 15 may be a single unit, or each robot may have its own separate control unit, interconnected by a third control module which interchanges and integrates the data from all separate controllers. But regardless of the arrangement used, the control functionality is configured such that the pose of all of the robotic actuating arms are known relative to each other. The controller is thus configured to know the position and orientation of each robotic arm in three-dimensional space. The first robotic arm 11 in the exemplary implementation shown in Fig. 1, in communication with and controlled by the controller 15, directs an endoscope 12 containing ports which are the end effectors for an imaging device 18 and for an irrigation device 19. The second robotic arm 13 operates at least one surgical end effector 14 such as a cautery element, drill, scalpel, or other surgical tool, and is also actuated by the controller 15. The data provided by the controller 15 can be viewed on a viewing monitor 17, which may be positioned as a unit with the controller 15, or may be configured as a separate unit which can be positioned so that the surgeon has easy view thereof.

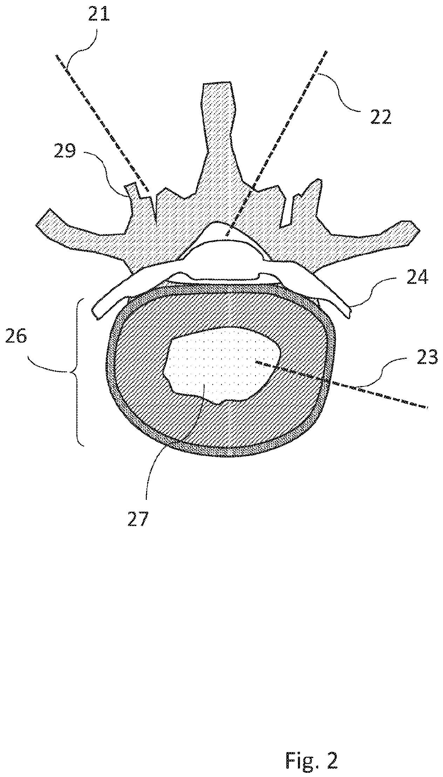

Reference is now made to Fig. 2, illustrating major aspects of spinal anatomy which can be accessed during a typical endoscopic spinal procedure, and three typical endoscopic approaches in some embodiments of the methods possible using the system of Fig. 1. The indication could be any of several types of surgical treatment, such as laminectomy for spinal decompression, flavectomy for spinal stenosis, nerve ablation for facet joint pain, foraminotomy for foraminal stenosis, or herniated disc removal to decompress a spinal nerve root. The vertebral body 26 is bounded by intervertebral discs 27 located in directions perpendicularly to the plane of the vertebral body, above and below the vertebral body. Herniation of the disc 27 may result in compression of the neural tissue, either the spinal cord or a nerve root 24. Three typical approach trajectories for the endoscopic port having individual channels for the camera and the irrigation device are shown in Fig. 1 as dashed lines: a peri-articular approach 21 to the facet joint, an epidural approach 22 to the spinal canal 22, and an intra-discal approach 23 to the intervertebral disc. Each of these approaches is suitable for a specific type or types of operative procedure. In each position, a working channel or cavity has to be generated by the irrigation device located in a channel within the endoscopic probe.

In order to best use the approaches described in Fig. 2, or any other suitable surgical procedure, the positions of the endoscopic port carrying the camera and irrigation device should be limited to areas that are between tissue planes, and thus, can hold fluid under pressure. For example, in an epidural approach 22, the endoscope may be inserted via an opening in the vertebral lamina into the epidural space. The epidural space is constrained by the meninges covering the neural tissue on the medial aspect, and the ligamentum flavum and vertebral processes on the lateral and posterior aspects. Thus, the injected fluid is partially confined to a naturally-occurring potential inter-tissue space, and fluid leakage should therefore be less than if the channel or cavity were made within a tissue lacking clear capsular or connective tissue boundaries. Another example of a tissue track would be that created by inserting the endoscope into an intervertebral disc for monitoring of a herniated disc removal. The intervertebral disc is surrounded by layers of connective tissue, thus confining the injected fluid within the intervertebral body. In a typical implementation, the pressure of the fluid injected by the irrigation device may be around 50 mmHg. The exact pressure will depend on the individual patient physiological parameters. The pressure typically will be great enough to hold the tissue planes apart such that a cavity is generated and maintained, but not higher than the diastolic blood pressure, since a higher pressure would compromise blood flow to the surrounding tissue. In some embodiments, the injected fluid may comprise 0.9% sterile saline below body temperature to reduce blood entry into the cavity.

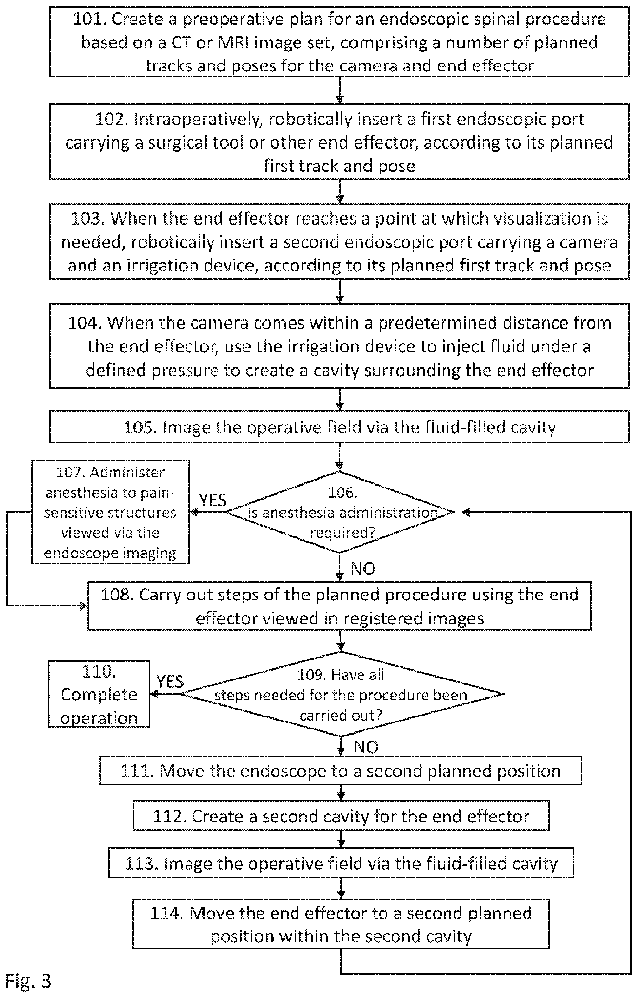

Reference is now made to Fig. 3, in which steps of a typical implementation of the methods enabled by embodiments of the present disclosure, are illustrated. In step 101, the surgeon creates a preoperative plan for an endoscopic spinal surgical procedure based on a three-dimensional CT or MRI or other imaging modality. The medical images may be subjected to segmentation, in order to automatically or semi-automatically identify boundaries such as tissue planes and borders between anatomical structures. Based on the segmentation analysis, a map of the surgical area of interest is generated, with annotation of specific anatomical structures in the area, such as selected aspects of vertebrae, intervertebral discs, neural tissue, connective tissue, nerves, and blood vessels. The specific anatomical structures may include both targets of the surgical procedure and structures to be avoided. This map may then be stored in the memory of the controller and used to compare with endoscopic images generated in subsequent steps.

Step 101 further comprises the steps of a plan for a specific operation, for example, discectomy, in an endoscopic manner. The plan may comprise numerous steps required to carry out the procedure. In each step of the procedure, the plan may comprise directions for the track to be followed by the endoscopic port carrying the surgical end effector under robotic control, in order to reach the pose of the surgical end effector required for each surgical act. The plan should also comprise the projected track for the endoscopic port with the camera and irrigation device, that pose being largely decided by the need for the camera to provide the optimum field of view. Other details of step 101, including the desired size of the cavity to be generated by the irrigation inflow, should also be decided. As the coordinate systems of the two (or more) robotic arms are known to the control system, the track of the end effector with the camera is calculated such that, during the segments of the procedure in which the camera is to provide visualization of the operation field, both the surgical end effector and the camera end effector will be positioned within a predetermined distance from each other. This distance may depend on a number of factors, including the required size of the fluid-filled cavity, the tissue in which the cavity is created, the type of camera, the surgical tool used, and any other relevant characteristic. In typical spinal surgery applications, the camera and surgical tool are kept within 1 to 2 cm of each other.

The calculated path to be taken by the endoscope port containing the camera is based on the intended goal of the operation and targets several criteria: 1) avoiding critical structures that could be damaged by the camera end effector passing through them; 2) not interfering with the position of the surgical end effector, 3) being able to be positioned such that the camera of this end effector is capable of viewing, after fluid injection, the surgical field and the end effector held by another robotic arm; and 4) the calculated path preferably follows tissue planes and minimizes trauma to the soft tissues along the path.

Whereas the general procedure is known ahead of time, each patient has different anatomical variations. Especially in individuals requiring surgical intervention, pathological changes in the bony anatomy may result in unexpected intraoperative findings, thus leading to challenges in navigation of the endoscopic arms within the patient. Therefore, the system is programmed for flexibility, in that the preoperative plan may be altered during the procedure based on unexpected findings encountered by the camera end effector, or based on images acquired in later steps of the procedure, such as in steps 108 and 111, to be discussed below.

Intraoperatively, in step 102, a first endoscopic port carrying a surgical tool or other surgical end effector is robotically inserted, according to the planned first track and pose. The tool is inserted up to a known depth where insertion is considered safe without reliance on direct or derived vision.

In step 103, when the surgical end effector reaches a point at which visualization is needed, a second endoscopic port carrying a camera and an irrigation device is robotically inserted, according to a preoperatively planned track and pose. The controller manipulates the second robotic arm containing the endoscopic camera and irrigation device, according to the preoperatively planned track to create a cavity. The cavity is created using a stream of fluid, such as sterile saline, fed through an irrigation device carried by the endoscope. In a typical embodiment, the irrigation device is a channel within the same endoscopic port that houses the channel carrying the camera. The volume and pressure of the injected fluid is calculated and adjusted such that the resulting fluid dynamics form a working channel or cavity in the tissue without causing excessive damage to the tissue.

In step 104, when the camera comes within a predetermined distance from the surgical end effector, the irrigation device is used to inject fluid under a defined pressure to create a cavity surrounding the surgical end effector. The predetermined distance is calculated by the controller according to the preoperative plan. The controller effectively takes the place of the surgeon's direct vision and hand-eye coordination, by storing in memory the planned movements of each robotic arm, and tracking in real time the coordinates of each tool, end effector, or camera as it is moved. The two endoscopic ports, under the control of separate robotic arms, are inserted via separate entrance points into the patient with the tip of each port aimed at the same general target region such that they thus become positioned in close proximity to each other. Once the cavity is created, the tip of the surgical end effector becomes visible to the camera within the cavity created by the irrigation device. Generally, the surgical end effector is inserted from one direction and the endoscope from the other, such that the endoscope creates a fluid-filled cavity in the vicinity of the surgical tool and provides visualization for the tool's robotically-controlled movements. The tracks of each element, the camera endoscope and the tool, are coordinated by the controller to create a coordinated interplay of the movements.

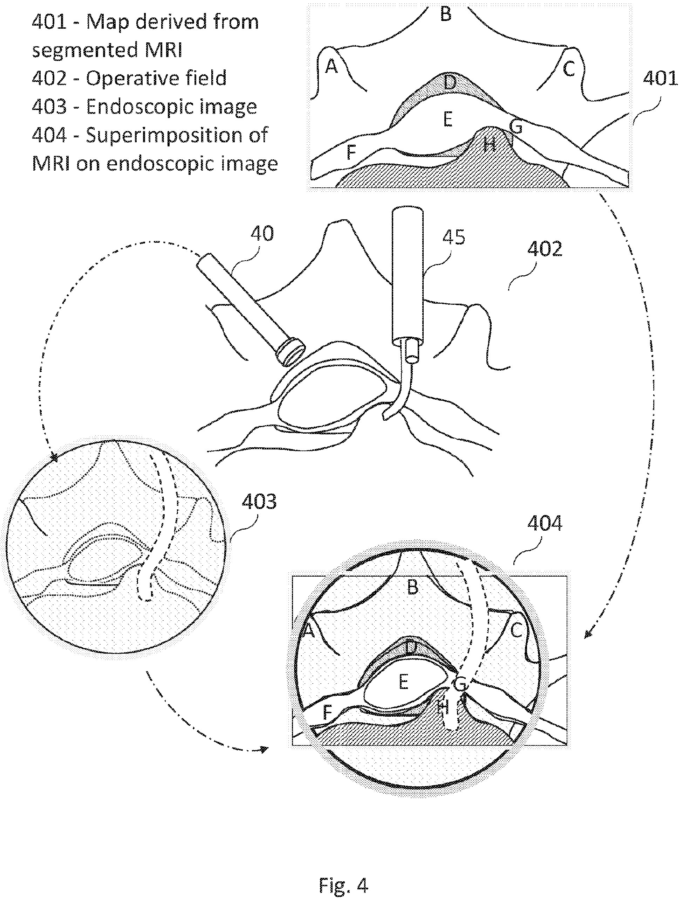

In step 105, once a working cavity is created, the camera, or another imaging device, is able to acquire surface images of the operative field in real time. These images create a field of vision, such that the camera images of surfaces surrounding the fluid-filled cavity enable the surgeon to observe the surgical tool, or other end effector, in its anatomic surroundings, and to view its progress in carrying out the surgical procedure. The surgeon may also interrupt the preplanned programming by instructing the controller to alter the actions of the tool or position of the camera. The imaging device held by the second robotic arm is used to provide real time images of the surgical field, and of the planned track of motion of that second working channel. The system calculates the optimal position and orientation from which the endoscopic camera can view the surgical field, and it interprets the image observed using its anatomical positioning program. The anatomical positioning may be accomplished in any number of separate ways, further explained in Fig. 4 below.

In step 106, the system determines if an injection of anesthesia is needed to the area under operation or to surrounding pain-sensitive regions. Such areas may be the surfaces of bones, known to have innervation and to be painfully sensitive to surgical trauma, or other areas. In some implementations, anesthesia administration is based on known pain-generating structures, like bone surfaces. The bony surface (periosteum) is a pain-sensitive structure. Local anesthetic should be applied to bony surfaces before drilling or scraping. In the same or other implementations, administration of anesthesia may be based on monitoring of patient vital signs. A predetermined rise in the patient's heart rate or blood pressure may trigger transmission of a signal indicating that a pain-inducing step may be taking place. Upon receiving such an alert, the system or the surgeon may decide to administer an additional dose of local anesthetic.

Once the system determines, either by preplanning, or by identification in the intraoperative images of a pain-sensitive structure, or by a measured change in the patient's physiological parameters, that anesthetic administration is required, the method proceeds to step 107, in which anesthesia is injected. A robotic arm, typically the arm carrying the surgical end effector, is used to administer anesthetic to pain-sensitive surfaces or regions. In other embodiments, the anesthetic may be injected via a channel in the endoscopic arm carrying the camera. In other cases, a third robotic arm may be employed to hold a syringe for administering anesthesia, or to hold another end effector. The anatomical region to be injected may be identified either from the preoperative images, or from the endoscopic images intraoperatively, or by direct vision by the surgeon of the anatomical structure to be anesthetized.

In step 108, the system manages the position and orientation, i.e., the pose, of the endoscopic camera to follow the movement of the surgical end effector, such that the surgical end effector or tool is held within view of the camera. The camera may project images in real time to a viewing screen so that the surgeon may be constantly updated as to the progress of the surgical procedure when a pre-planned step is being carried out. For each step of the procedure, any time a) a new static position of the endoscopic camera in a new cavity position is reached, or b) the surgeon is unsure of the identity of an anatomical structure within the cavity, an image of the newly-produced cavity may be acquired before any further actions by the end effector are performed. The updated intraoperative image may be registered with the preoperative images to provide an updated map of the anatomical structures within the cavity. This repeated updating of the registration process is a safety feature that ensures that the surgeon is constantly notified of the anatomical features in the surgical field. The time taken for this registration process usually more than compensates for time spent in exploratory maneuvers because of uncertainty of what is being observed, and enables better outcomes as structures are clearly identifiable.

At some point in the procedure, the surgeon may decide to take control of the operation. It is difficult to predict preoperatively how much tissue must be removed, for example, to release pressure on a nerve. Thus, the surgeon needs to make decisions on how to proceed, and provides instructions to the controller of the surgical robot, which then carries out the steps as directed by the surgeon. At an appropriate point of the operation, for example toward the end of the procedure when the closing steps are more standardized, the surgeon may opt to return control of the procedure to the system, which automatically calculates and executes a sequence of positions for continuing or completing the procedure.

In step 109, the system assesses whether all of the steps have been carried out to complete the planned procedure. If so, step 110 is reached, in which the operation is completed by the surgeon or the robotic system.

On the other hand, if in step 109, it is determined that not all of the required surgical steps have been carried out, in step 111, the endoscope carrying the camera is moved to a second or additional position to enable the system to continue carrying out the next steps of the procedure. This position may have been planned previously as part of the preoperative plan, or may be decided intraoperatively by the surgeon, based on intraoperative findings. Typically, the initial steps of the procedure are pre-planned and carried out under robotic control. Intraoperatively, once the operative field is opened and the pathology to be surgically corrected is identified in the images acquired by the endoscopic camera, the subsequent steps of the procedure may be carried out according to the preoperative plan, or may be determined or altered by the surgeon. In most cases, it is difficult to plan each step of the procedure preoperatively; thus, the option exists for the surgeon to modify or change the track and pose of both the endoscopic camera and the surgical end effector at any point during the procedure.

In step 112, after the endoscope is moved to a subsequent position to enable further steps of the procedure to be performed, a second fluid-filled cavity is created for the end effector. The second position is typically located along a continuation of the initial track. For each position of the camera endoscope and the surgical end effector, the system calculates the size and position of a cavity to be created, and then creates it by injecting fluid between tissue planes or within a region which is recognized as a potential cavity. In some embodiments, the cavity may be 1 to 2 cm in diameter, and should be large enough to accommodate the surgical end effector and to image the field as the end effector is manipulated within the cavity.

In step 113, the camera acquires updated images within the second or subsequent fluid-filled cavity. At some points of the procedure, as for example when the end effector is performing an action, the camera may obtain and the system may display real time images of the surgical field. However, before a surgical step is carried out, a static image of the field of view in the cavity is compared with the expected image, based on the preoperative CT or MRI images. The view of the surgical field in images generated by the endoscopic camera will be different from the view obtained by looking directly at the surgical field in an open operation, because the pressure of the fluid filling the cavity creates a spherical distortion of the three-dimensional space. Thus, typical landmarks may be missing from the view, and the orientation of the anatomical structures are also altered, making identification of structures a challenge. In some embodiments, the endoscopic image is thus annotated with a map derived in step 101 from segmentation of the MRI or CT images, as further explained with respect to Fig. 4 below.

In step 114, the end effector is robotically moved to a subsequent position within the cavity created in step 112. The positioning of the end effector is performed according to the registered, annotated image acquired in step 113. The position of anatomical features of interest is known from the annotation on the registered endoscopic camera image, even if they are not visually identifiable by the surgeon. Thus, the surgeon is able to instruct the robotic controller to manipulate the end effector based on the features identified in the intraoperative image. As the coordinate systems of both robotic arms are known to the system controller, the end effector may be safely manipulated under robotic control using instructions from the surgeon. The end effector is moved into position within the fluid cavity, and can then perform steps of the operation under direct visualization. In any given position of the cavity and the end effector, the surgeon may carry out a stage of the operation. When the possible maneuvers have been completed at that position, the procedure will require a new position of the camera and end effector. At this point, the method returns to step 106 to determine whether injection of additional anesthetic is required. The loop of steps from 106-114 is performed as many times as necessary, for some operations between 10 to 20 times, each time completing a little more of the complete required procedure, until all of the steps required for a successful completion of the operation have been performed.

Reference is now made to Fig. 4, illustrating schematically the manner in which different views of the operative region are obtained and superimposed. In step 401, the preoperative CT or MRI is obtained and annotated with identification of specific anatomical structures, represented in this example by letters A-H. Lettered structures correspond to features of the spinal column, identified as follows. A, C - vertebral facets; B - vertebral spinous process; D - epidural space; E - spinal cord; F - dorsal root ganglion; G - nerve roots; H - herniated disc. These features are shown for the purpose of illustration; for each patient, anatomical features will need to be identified based on the region of interest and the specific pathology to be treated.

Intraoperatively, as shown in step 402, the endoscopic camera 40 obtains a view of the operative field, which is schematically shown in 403. The camera 40 may be positioned such that it images the surgical end effector 45. Since the camera image is a small, two-dimensional surface view of internal tissue surfaces, the view provided by the endoscopic camera is less clear than the density-weighted MRI or CT images. Additionally, the image may be distorted by the fluid pressure used to create the cavity. The track of the end effector, as seen in step 403, is shown in dashed outline.

In step 404, the intraoperative image is registered to the preoperative image set. By registering the camera image of the surgical field to the preoperative, segmented and annotated MRI or CT images, the surgeon is able to identify specific anatomical structures in the operative field that would be unclear or unidentifiable in the endoscopic images. After the registration process is completed, the identified features from the MRI or CT may be labeled on a virtual representation of the camera image on a screen or monitor. With image registration, each pixel in a two-dimensional camera image corresponds to a voxel in the MRI or CT image. The three-dimensional MRI or CT image set can be virtually sliced or rotated to find the angle that corresponds to the intraoperative camera image. The derived two-dimensional MRI or CT image, representing the expected view of the imaged region, is then compared with the camera image, which represents the real life view. Due to differences in the tissue arrangements in the preoperative anatomy compared with the intraoperative anatomy, the images often do not align precisely. Thus, image processing may be used to update the registration to result in accurate alignment. The addition of image registration and annotation provides a greater degree of accuracy than would be possible without having a preoperative image of the operative field. The expected error of the robotic system without image registration and identification of anatomical features is in the range of up to 2mm. By adding registration of the endoscopic camera image with the preoperative MRI, in which features are more easily identifiable, the resolution of the procedure may be improved to 0.1mm, which is well within the currently acceptable margin of error for surgical procedures performed by a surgeon.

The image registration may be accomplished in several ways. One way is as described above, by alignment of the two-dimensional intraoperative image representation of the three-dimensional surgical field, with the three-dimensional preoperative images. Even though the camera images will have been acquired at a different angle from the preoperative CT or MRI images, image processing algorithms can be used to virtually slice through the three-dimensional images at any selected angle to produce a two-dimensional image corresponding to the surface viewed by the endoscopic camera. The selected angle for virtual slicing may be determined by alignment of the preoperative images with a registration performed at the outset of the procedure. In other embodiments, the angle for virtual slicing of the CT or MRI images may be determined by recognition of specific anatomical features in both the preoperative images and in the camera-acquired intraoperative images. The anatomical positioning component of the registration comprises feature recognition, which may be performed using image processing supported by machine learning or other artificial intelligence means, such that the same anatomical structure can be identified in two separate imaging modalities. The method aligns several anatomical features in the images acquired preoperative and intraoperatively, such that it becomes possible to overlay the intraoperative image, comprising the derived field of view, on an equivalent two-dimensional plane of the three-dimensional preoperative images with anatomical features identified. The feature identification enables the surgeon to know the location of the surgical tool with respect to identified anatomical features, thus generating a labeled virtual map of the surgical field.

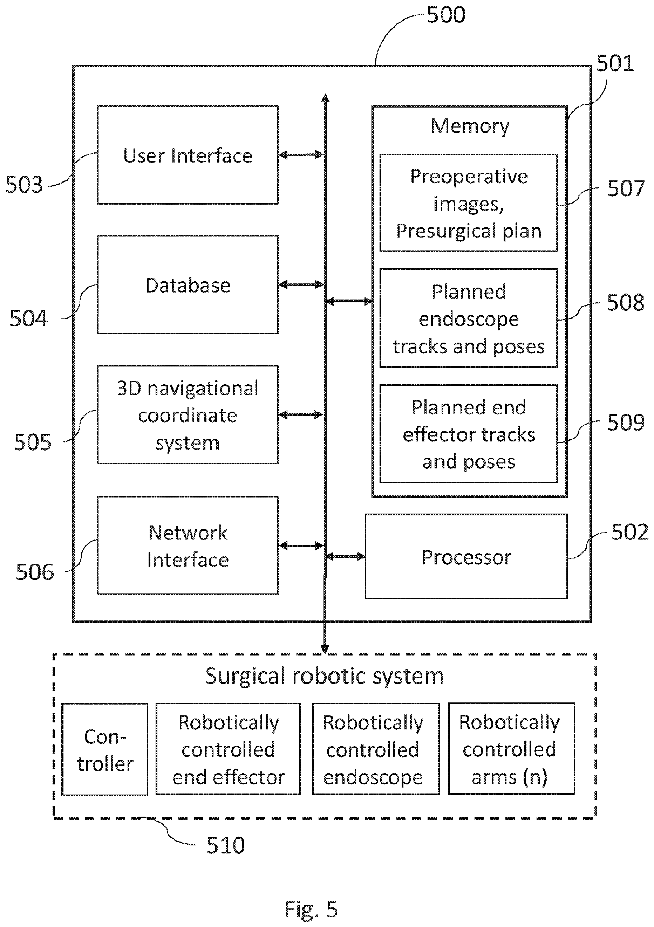

Reference is now made to Fig. 5, illustrating in a schematic block diagram the structures used for carrying out some embodiments of the system of the present disclosure. The surgical robotic system 510 comprises a controller and two or more robotically controlled arms, configured to hold and carry an endoscopic camera, surgical end effector, or other relevant element needed for the operation. The robotic system is in communication with a control system 500 encompassing the claimed system and having a number of components. These components comprise a processor 502, a network interface 506, a user interface 503 and a memory 501. Optionally, the system may comprise a three-dimensional navigational system 505, and a database 504 containing at least results of prior operations, image processing rules and algorithms, annotated images of normal anatomical structures in a variety of imaging modalities, or all of the above. In a typical embodiment, the memory 501 contains at least the pre-surgical plan 507 based on the preoperative three-dimensional images, calculated endoscope tracks and poses 508, and planned end effector tracks and poses 509. Embodiments of the disclosed control system 500 are connected to or in communication with the surgical robotic system 510, which incorporates a controller and typically a number (n) of robotically controlled arms enabled to carry one or more end effectors, an anesthetic injection device, a camera, and an irrigation device. The controller of the robotic system 510 controls the coordinates of each robotic arm, such that the position of the end effector and the endoscope are able to be used and manipulated in tandem without direct visualization.

## Claims

1. A system (10) for endoscopic surgery, comprising:
a first robotic arm (11) configured to define the pose of an endoscopic camera (12) carried thereon;
a second robotic arm (13) configured to define the pose of a surgical end effector (14) carried thereon;
an irrigation nozzle configured to inject fluid into a preselected tissue region, generating a cavity in a locality where a surgical procedure is to be performed; and
at least one controller (15) configured to control at least the first robotic arm (11) and the second robotic arm (13), such that the pose of the endoscopic camera (12) and the pose of the surgical end effector (14) are known to the controller (15),
wherein the endoscopic camera (12) is configured to acquire intraoperative images of the cavity, such that an endoscopic operation can be performed in the cavity with the surgical end effector (14) guided according to the intraoperative images of the cavity,
**characterized in that** the fluid is injected under a predetermined pressure, below a diastolic blood pressure of a subject on whom the endoscopic surgery is being performed, and
**in that** the irrigation nozzle is incorporated into an endoscopic element carried by the first robotic arm (11).

2. The system (10) according to claim 1, wherein the at least one controller (15) is configured to control the first robotic arm (11) to adjust the pose of the endoscopic camera (12) such that it views the surgical end effector (14) whose position is adjusted by the second robotic arm (13).

3. The system (10) according to claim 1, further comprising a third robotic arm configurable to administer a drug to a surgical field.

4. The system (10) according to claim 3, wherein the drug is an anesthetic agent.

5. The system (10) according to claim 1, wherein the at least one controller (15) is configured to perform steps necessary to carry out a surgical procedure using the second robotic arm (13).

6. The system (10) according to claim 1, wherein the endoscopic operation is capable of being performed minimally invasively.

7. The system (10) according to claim 1, wherein the endoscopic operation is any one of a disc herniectomy, a laminotomy, a laminectomy, facet joint nerve ablation, or a foraminotomy.

8. The system (10) according to claim 1, wherein the preselected tissue region is a space between tissue planes.

9. The system (10) according to claim 1, wherein the preselected tissue region may comprise the inside of an intervertebral disc, an epidural space, or fascial layers between paraspinal muscles.

10. The system (10) according to claim 1, wherein the surgical end effector (14) comprises any of a drill, a scalpel, a bone scraper, a rongeur, a suction device, an ablation head, a cautery, forceps, or another spinal surgical tool.

## Patentansprüche

1. System (10) für endoskopische Chirurgie, umfassend:
einen ersten Roboterarm (11), der konfiguriert ist, um die Haltung einer endoskopischen Kamera (12), die darauf getragen wird, zu definieren;
einen zweiten Roboterarm (13), der konfiguriert ist, um die Haltung eines chirurgischen Endeffektors (14), der darauf getragen wird, zu definieren;
eine Spüldüse, die konfiguriert ist, um Fluid in einen vorab ausgewählten Gewebebereich zu injizieren, wobei an einer Stelle, an der ein chirurgischer Eingriff durchgeführt werden soll, eine Kavität erzeugt wird; und
mindestens eine Steuereinrichtung (15), die konfiguriert ist, um mindestens den ersten Roboterarm (11) und den zweiten Roboterarm (13) derart zu steuern, dass die Haltung der endoskopischen Kamera (12) und die Haltung des chirurgischen Endeffektors (14) der Steuereinrichtung (15) bekannt sind,
wobei die endoskopische Kamera (12) konfiguriert ist, um intraoperative Bilder der Kavität derart zu erfassen, dass eine endoskopische Operation in der Kavität durchgeführt werden kann, wobei der chirurgische Endeffektor (14) gemäß den intraoperativen Bildern der Kavität geführt wird,
**dadurch gekennzeichnet, dass** das Fluid unter einem zuvor bestimmten Druck injiziert wird, der unter einem diastolischen Blutdruck eines Subjekts, an dem die endoskopische Chirurgie durchgeführt wird, liegt, und
**dass** die Spüldüse in ein endoskopisches Element, das durch den ersten Roboterarm (11) getragen wird, integriert ist.

2. System (10) nach Anspruch 1, wobei die mindestens eine Steuereinrichtung (15) konfiguriert ist, um den ersten Roboterarm (11) zu steuern, um die Haltung der endoskopischen Kamera (12) derart anzupassen, dass sie den chirurgischen Endeffektor (14) betrachtet, dessen Position durch den zweiten Roboterarm (13) angepasst wird.

3. System (10) nach Anspruch 1, ferner umfassend einen dritten Roboterarm, der konfigurierbar ist, um einem Operationsfeld ein Arzneimittel zu verabreichen.

4. System (10) nach Anspruch 3, wobei das Arzneimittel ein Anästhetikum ist.

5. System (10) nach Anspruch 1, wobei die mindestens eine Steuereinrichtung (15) konfiguriert ist, um Schritte durchzuführen, die nötig sind, um einen chirurgischen Eingriff unter Verwendung des zweiten Roboterarms (13) auszuführen.

6. System (10) nach Anspruch 1, wobei die endoskopische Operation minimalinvasiv durchgeführt werden kann.

7. System (10) nach Anspruch 1, wobei die endoskopische Operation eine beliebige von einer Bandscheibenherniektomie, einer Laminotomie, einer Laminektomie, einer Facettengelenknervenablation oder eine Foraminotomie ist.

8. System (10) nach Anspruch 1, wobei der vorab ausgewählte Gewebebereich ein Raum zwischen Gewebeebenen ist.

9. System (10) nach Anspruch 1, wobei die vorab ausgewählte Gewebebereich die Innenseite einer Bandscheibe, einen Epiduralraum oder Faszienschichten zwischen paraspinalen Muskeln umfassen kann.

10. System (10) nach Anspruch 1, wobei der chirurgische Endeffektor (14) ein beliebiges von einem Bohrer, einem Skalpell, einen Knochenschaber, einem Rongeur, einer Abaugvorrichtung, einem Ablationskopf, einem Kauter, einer Zange oder einem anderen spinalen chirurgischen Werkzeug umfasst.

## Revendications

1. Système (10) pour chirurgie endoscopique, comprenant :
un premier bras robotique (11) configuré pour définir la position d'une caméra endoscopique (12) montée sur celui-ci ;
un deuxième bras robotique (13) configuré pour définir la position d'un effecteur terminal chirurgical (14) monté sur celui-ci ;
une buse d'irrigation conçue pour injecter un fluide dans une région tissulaire présélectionnée, générant une cavité dans une localité où une intervention chirurgicale doit être effectuée ; et
au moins un dispositif de commande (15) configuré pour commander au moins le premier bras robotique (11) et le deuxième bras robotique (13), de telle sorte que la position de la caméra endoscopique (12) et la position de l'effecteur terminal chirurgical (14) sont connues du dispositif de commande (15),
dans lequel la caméra endoscopique (12) est configurée pour acquérir des images peropératoires de la cavité, de telle sorte qu'une opération endoscopique puisse être effectuée dans la cavité avec l'effecteur terminal chirurgical (14) guidé selon les images peropératoires de la cavité,
**caractérisé en ce que** le fluide est injecté sous une pression prédéterminée, inférieure à une pression sanguine diastolique d'un sujet sur lequel la chirurgie endoscopique est effectuée, et
**en ce que** la buse d'irrigation est incorporée dans un élément endoscopique porté par le premier bras robotique (11).

2. Système (10) selon la revendication 1, dans lequel l'au moins un dispositif de commande (15) est configuré pour commander le premier bras robotique (11) afin d'ajuster la position de la caméra endoscopique (12) de telle sorte qu'elle voie l'effecteur terminal chirurgical (14) dont la position est ajustée par le deuxième bras robotique (13).

3. Système (10) selon la revendication 1, comprenant en outre un troisième bras robotique configurable pour administrer un médicament à un champ chirurgical.

4. Système (10) selon la revendication 3, dans lequel le médicament est un agent anesthésique.

5. Système (10) selon la revendication 1, dans lequel l'au moins un dispositif de commande (15) est configuré pour effectuer les étapes nécessaires à la réalisation d'une intervention chirurgicale à l'aide du deuxième bras robotique (13).

6. Système (10) selon la revendication 1, dans lequel l'opération endoscopique peut être effectuée de manière mini-invasive.

7. Système (10) selon la revendication 1, dans lequel l'opération endoscopique est l'une quelconque parmi hernie discale, laminotomie, laminectomie, ablation du nerf de l'articulation facettaire ou foraminotomie.

8. Système (10) selon la revendication 1, dans lequel la région tissulaire présélectionnée est un espace entre les plans tissulaires.

9. Système (10) selon la revendication 1, dans lequel la région tissulaire présélectionnée peut comprendre l'intérieur d'un disque intervertébral, un espace épidural ou des couches fasciales entre les muscles paraspinaux.

10. Système (10) selon la revendication 1, dans lequel l'effecteur terminal chirurgical (14) comprend l'un quelconque parmi foret, scalpel, racleur d'os, éponge, dispositif d'aspiration, tête d'ablation, cautère, pince ou autre outil chirurgical pour la colonne vertébrale.
